# EUROPEAN PATENT APPLICATION

(11) **EP 3 438 265 A1**
(43) Date of publication of application: **06.02.2019**
(21) Application number: 17184522.5
(22) Date of filing: 02.08.2017
(51) Int. Cl.: C12N 15/113, C12N 9/12, A61P 35/00

(54) **DUAL INHIBITION OF IKK1 AND IKK2 FOR THE TREATMENT OF PROLIFERATIVE DISEASES**

(71) Applicant: Universität Bielefeld, 33615 Bielefeld (DE)
(72) Inventor: Kaltschmidt, Barbara, 33619 Bielefeld (DE); Kaltschmidt, Christian, 33619 Bielefeld (DE); Storm, Jonathan, 33613 Bielefeld (DE); Slotta, Carsten, 33619 Bielefeld (DE); Greiner, Johannes, 33615 Bielefeld (DE)
(74) Representative: Kuttenkeuler, David

(57) **Abstract**

The present invention pertains to dual inhibition of Inhibitor of κB Kinase (IKK) 1 and IKK2 in a biological cell for the treatment of tumors. The invention provides compositions comprising IKK1 and IKK2 antagonists for use in medical treatments of proliferative disorders, in particular for the treatment of cancer. Most preferred in context of the invention is a gene editing approach for a targeted simultaneous knock out of the genes of IKK1 and IKK2 in a cell associated with the proliferative disorder.

## Description

### FIELD OF THE INVENTION

The present invention pertains to dual inhibition of Inhibitor of κB Kinase (IKK) 1 and IKK2 in a biological cell for the treatment of tumors. The invention provides compositions comprising IKK1 and IKK2 antagonists for use in medical treatments of proliferative disorders, in particular for the treatment of cancer. Most preferred in context of the invention is a gene editing approach for a targeted simultaneous knock out of the genes of IKK1 and IKK2 in a cell associated with the proliferative disorder.

### DESCRIPTION

The transcription factor Nuclear Factor kappa-B (NF-κB) is expressed in numerous cell types in which it functions as a master regulator of proliferative, immune and inflammatory responses. In unstimulated cells, NF-κB is complexed with Inhibitor of kappa-B (IκB) proteins in an inactive state. Upon stimulation, IκB is phosphorylated by IκB-Kinase (IKK) which leads to ubiquitination and subsequent degradation of IκB by the proteasome pathway. Loss of IκB exposes a nuclear translocation signal on NF-κB, allowing the transcription factor to enter the nucleus and bind to promoter response elements of NF-κB responsive genes. These NF-κB activated genes include a wide array of inflammatory mediators such as cytokines, chemokines, adhesion molecules, growth factors, and inflammatory enzymes.

There are two main pathways by which NF-κB can be activated: the canonical pathway and the non-canonical pathway. IKK2 is the key regulatory enzyme in the canonical or classical pathway. IKK2 for example is activated by proinflammatory stimuli which leads to phosphorylation of IκB and its subsequent degradation. It is widely accepted that the canonical pathway is responsible for NF-κB activation in inflammatory states and that inhibition of IKK2 is sufficient to block the majority of NF-κB induced inflammation. The non-canonical or alternative pathway involves activation of IκB kinase 1 (IKK1), which phosphorylates p100 (NF-κB2), subsequently releasing RelB to form transcriptionally active p52-RelB heterodimers. IKK1 is required for secondary lymphoid organogenesis and B cell maturation and survival. IKK1 does also contribute to inflammatory resolution. Thus, a method to inhibit simultaneously IKK1 and IKK2 rather than either alone has surprising advantages for the treatment of proliferative diseases.

WO/1998/008955 discloses compositions and methods for treating NF-κB -related conditions. In particular, the disclosure provides a stimulus-inducible IKK signalsome, and components and variants thereof that are useful for therapy of conditions such as cancer.

Due to the general need in the art for new and effective cancer therapeutics it was a primary object to provide new options for the therapy of proliferative diseases.

The above problem is solved in a first aspect by a method for treating a proliferative disorder in a subject, the method comprising the step of reducing or impairing the function or expression of both IKK1 and IKK2 simultaneously in a cell which is associated with the proliferative disorder in the subject. Alternatively, the first aspect of the invention then pertains to a compound for use in this method, wherein the compound is necessary for reducing or impairing the function or expression of both IKK1 and IKK2 simultaneously in a cell which is associated with the proliferative disorder in the subject. Such a compound could be an antagonist or inhibitor of IKK1 and/or IKK2, or a composition of antagonists or inhibitors of IKK1 and/or IKK2.

In context of the present invention it was surprisingly discovered that dual inactivation or inhibition of IKK1 and IKK2 in a tumor cell leads to a strong induction of apoptosis in the presence of the signaling factor tumor necrosis factor (TNF)-α. Using the targeted approach for inhibition of IKK1 and IKK2, preferably on the genome level, therefore opens up new therapeutic avenues to tackle proliferative disorders such as cancer. Hence, the present methods and compositions provide a means for the selective induction of apoptosis in cells associated with a proliferative disorder, such as tumor cells.

Therefore, in a preferred embodiment of the first aspect of the invention the proliferative disorder is a tumor disease, such as ovarian cancer, and said cell associated with the proliferative disorder in the subject is a tumor cell. The terms "tumor disease" or "cancer" shall are used synonymously in the following and may include, but are not limited to, leukemias such as chronic lymphocytic leukemia, chronic myelocytic leukemia, acute lymphoblastic leukemia, acute myelogenous leukemia, and acute myeloblastic leukemia; advanced malignancy, amyloidosis, neuroblastoma, meningioma, hemangiopericytoma, multiple brain metastase, glioblastoma multiforms, glioblastoma, brain stem glioma, poor prognosis malignant brain tumor, malignant glioma, recurrent malignant giolma, anaplastic astrocytoma, anaplastic oligodendroglioma, neuroendocrine tumor, rectal adenocarcinoma, Dukes C & D colorectal cancer, unresectable colorectal carcinoma, metastatic hepatocellular carcinoma, Kaposi's sarcoma, karotype acute myeloblastic leukemia, Hodgkin's lymphoma, non-Hodgkin's lymphoma, cutaneous T-Cell lymphoma, cutaneous B-Cell lymphoma, diffuse large B-Cell lymphoma, low grade follicular lymphoma, malignant melanoma, malignant mesothelioma, malignant pleural effusion mesothelioma syndrome, peritoneal carcinoma, papillary serous carcinoma, gynecologic sarcoma, soft tissue sarcoma, scleroderma, cutaneous vasculitis, Langerhans cell histiocytosis, leiomyosarcoma, fibrodysplasia ossificans progressive, hormone refractory prostate cancer, resected high-risk soft tissue sarcoma, unrescectable hepatocellular carcinoma, Waldenstrom's macroglobulinemia, smoldering myeloma, indolent myeloma, fallopian tube cancer, androgen independent prostate cancer, androgen dependent stage 1V non-metastatic prostate cancer, hormone-insensitive prostate cancer, chemotherapy-insensitive prostate cancer, papillary thyroid carcinoma, follicular thyroid carcinoma, medullary thyroid carcinoma, and leiomyoma. In one embodiment, the cancer is primary or metastatic. In another embodiment, the cancer is relapsed, refractory or resistance to chemotherapy or radiation. Preferred cancer to be treated in accordance with the invention is ovarian cancer.

In context of the herein disclosed invention the term "subject" generally refers to any mammal. A subject of the invention is preferably a subject in need of a therapeutic treatment. The subject preferably is a subject suffering from a proliferative disorder. A subject of the invention in preferred embodiments is a primate, pet, mouse, rat or guinea pig, but preferably is a human subject, alternatively referred to herein as human patient.

In context of the herein disclosed invention the reducing or impairing the function or expression of both IKK1 and IKK2 simultaneously in a cell may be effected by any means available in the art. Preferably by introducing into the cell antagonists or inhibitors of IKK1 and/or IKK2. In context of the invention the terms "antagonist" and "inhibitor" shall be used interchangeably and refer to a substance that affects a decrease in the amount or rate of IKK expression or activity. Such a substance can act directly, for example, by binding to IKK and decreasing the amount or rate of IKK expression or activity. A IKK-antagonist can also decrease the amount or rate of IKK expression or activity, for example, by binding to IKK in such a way as to reduce or prevent interaction of IKK with a IKK substrate; by binding to IKK and modifying it, such as by removal or addition of a moiety; and by binding to IKK and reducing its stability. A IKK-antagonist can also act indirectly, for example, by binding to a regulatory molecule or gene region so as to modulate regulatory protein or gene region function and affect a decrease in the amount or rate of IKK expression or activity. Thus, a IKK-antagonist can act by any mechanisms that result in decrease in the amount or rate of IKK expression or activity.

A IKK-antagonist can be, for example, a naturally or non-naturally occurring macromolecule, such as a polypeptide, peptide, peptidomimetic, nucleic acid, carbohydrate or lipid. A IKK-antagonist further can be an antibody, or antigen-binding fragment thereof, such as a mono-clonal antibody, humanized antibody, chimeric antibody, minibody, bifunctional antibody, single chain antibody (scFv), variable region fragment (Fv or Fd), Fab or F(ab)2. A IKK-antagonist can also be polyclonal antibodies specific for IKK. A IKK-antagonist further can be a partially or completely synthetic derivative, analog or mimetic of a naturally occurring macromolecule, or a small organic or inorganic molecule.

A IKK-antagonist that is an antibody can be, for example, an antibody that binds to IKK and inhibits binding to a IKK receptor, or alters the activity of a molecule that regulates IKK expression or activity, such that the amount or rate of IKK expression or activity is decreased. An antibody useful in a method of the invention can be a naturally occurring antibody, including a monoclonal or polyclonal antibodies or fragment thereof, or a non-naturally occurring antibody, including but not limited to a single chain antibody, chimeric antibody, bifunctional antibody, complementarity determining region-grafted (CDR-grafted) antibody and humanized antibody or an antigen-binding fragment thereof.

A IKK-antagonist that is a nucleic acid can be, for example, an anti-sense nucleotide sequence, an RNA molecule, or an aptamer sequence. An anti-sense nucleotide sequence can bind to a nucleotide sequence within a cell and modulate the level of expression of IKK, or modulate expression of another gene that controls the expression or activity of IKK. Similarly, an RNA molecule, such as a catalytic ribozyme, can bind to and alter the expression of the IKK gene, or other gene that controls the expression or activity of IKK. An aptamer is a nucleic acid sequence that has a three dimensional structure capable of binding to a molecular target.

A IKK-antagonist that is a nucleic acid also can be a double-stranded RNA molecule for use in RNA interference methods. RNA interference (RNAi) is a process of sequence-specific gene silencing by post-transcriptional RNA degradation, which is initiated by double-stranded RNA (dsRNA) homologous in sequence to the silenced gene. A suitable double-stranded RNA (dsRNA) for RNAi contains sense and antisense strands of about 21 contiguous nucleotides corresponding to the gene to be targeted that form 19 RNA base pairs, leaving overhangs of two nucleotides at each 3' end (Elbashir et al., Nature 411:494-498 (2001); Bass, Nature 411:428-429 (2001); Zamore, Nat. Struct. Biol. 8:746-750 (2001)). dsRNAs of about 25-30 nucleotides have also been used successfully for RNAi (Karabinos et al., Proc. Natl. Acad. Sci. USA 98:7863-7868 (2001). dsRNA can be synthesized in vitro and introduced into a cell by methods known in the art.

In preferred embodiments an IKK antagonist is a compound used for targeted gene editing of an IKK encoding gene. Therefore, in this embodiment the method of the invention involves gene editing, gene silencing (RNA interference) or small molecule inhibitors of IKK1 and IKK2.

Preferably reducing or impairing the function or expression of both IKK1 and IKK2 simultaneously in a cell involves gene editing comprising the introduction of guide RNA(s) into the cell for targeting the genes of IKK1 and IKK2.

As used herein the term "gene editing" or genome editing refers to a type of genetic engineering in which DNA is inserted, replaced, or removed from a genome using artificially engineered nucleases, or "molecular scissors". The nucleases create specific double-strand breaks (DSBs) at desired locations in the genome, and harness the cell's endogenous mechanisms to repair the induced break by natural processes of homologous recombination (HR) and nonhomologous end-joining (NHEJ).

Methods described herein preferably take advantage of a CRISPR system. Therefore, in a preferred embodiment the antagonist of IKK (1 and/or 2) is preferably a component of a CRISPR mediated gene editing approach, and most preferably is a guide or sgRNA specific for IKK1 and/or IKK2). There are at least five types of CRISPR systems which all incorporate RNAs and Cas proteins. Types I, III, and IV assemble a multi-Cas protein complex that is capable of cleaving nucleic acids that are complementary to the crRNA. Types I and III both require pre-crRNA processing prior to assembling the processed crRNA into the multi-Cas protein complex. Types II and V CRISPR systems comprise a single Cas protein complexed with at least one guiding RNA.

The general mechanism and recent advances of CRISPR system is discussed in Cong, L. et al., "Multiplex genome engineering using CRISPR systems," Science, 339(6121): 819-823 (2013); Fu, Y. et al., "High-frequency off-target mutagenesis induced by CRISPR-Cas nucleases in human cells," Nature Biotechnology, 31, 822-826 (2013); Chu, VT et al. "Increasing the efficiency of homology- directed repair for CRISPR-Cas9-induced precise gene editing in mammalian cells," Nature Biotechnology 33, 543-548 (2015); Shmakov, S. et al, "Discovery and functional characterization of diverse Class 2 CRISPR-Cas systems," Molecular Cell, 60, 1-13 (2015); Makarova, KS et al, "An updated evolutionary classification of CRISPR-Cas systems,", Nature Reviews Microbiology, 13, 1- 15 (2015). Site-specific cleavage of a target DNA occurs at locations determined by both 1) base- pairing complementarity between the guide RNA and the target DNA (also called a protospacer) and 2) a short motif in the target DNA referred to as the protospacer adjacent motif (PAM). For example, an engineered cell can be generated using a CRISPR system, e.g., a type II CRISPR system. A Cas enzyme used in the methods disclosed herein can be Cas9, which catalyzes DNA cleavage.

Enzymatic action by Cas9 derived from *Streptococcus pyogenes* or any closely related Cas9 can generate double stranded breaks at target site sequences which hybridize to 20 nucleotides of a guide sequence and that have a protospacer-adjacent motif (PAM) following the 20 nucleotides of the target sequence.

A vector can be operably linked to an enzyme-coding sequence encoding a CRISPR enzyme, such as a Cas protein (CRISPR-associated protein). Non-limiting examples of Cas proteins can include Cas1, Cas1B, Cas2, Cas3, Cas4, Cas5, Cas6, Cas7, Cas8, Cas9 (also known as Csn1 or Csx12), Cas1O, Csy1, Csy2, Csy3, Cse1, Cse2, Csc1, Csc2, Csa5, Csn2, Csm2, Csm3, Csm4, Csm5, Csm6, Cmr1, Cmr3, Cmr4, Cmr5, Cmr6, Csb1, Csb2, Csb3, Csx17, Csx14, Csx1O, Csx16, CsaX, Csx3, Csx1, Csx1S, Csf1, Csf2, CsO, Csf4, Cpfl, c2c1, c2c3, Cas9HiFi, homologues thereof, or modified versions thereof. An unmodified CRISPR enzyme can have DNA cleavage activity, such as Cas9. A CRISPR enzyme can direct cleavage of one or both strands at a target sequence, such as within a target sequence and/or within a complement of a target sequence. For example, a CRISPR enzyme can direct cleavage of one or both strands within or within about 1, 2, 3, 4, 5, 6, 7, 8, 9, 10, 15, 20, 25, 50, 100, 200, 500, or more base pairs from the first or last nucleotide of a target sequence. A vector that encodes a CRISPR enzyme that is mutated with respect to a corresponding wild-type enzyme such that the mutated CRISPR enzyme lacks the ability to cleave one or both strands of a target polynucleotide containing a target sequence can be used. A Cas protein can be a high fidelity cas protein such as Cas9HiFi.

A vector that encodes a CRISPR enzyme comprising one or more nuclear localization sequences (NLSs), such as more than or more than about 1, 2, 3, 4, 5, 6, 7, 8, 9, 10, NLSs can be used. For example, a CRISPR enzyme can comprise more than or more than about 1, 2, 3, 4, 5, 6, 7, 8, 9, 10, NLSs at or near the ammo-terminus, more than or more than about 1, 2, 3, 4, 5, 6, 7, 8, 9, 10, NLSs at or near the carboxyl-terminus, or any combination of these (e.g. , one or more NLS at the ammo- terminus and one or more NLS at the carboxyl terminus). When more than one NLS is present, each can be selected independently of others, such that a single NLS can be present in more than one copy and/or in combination with one or more other NLSs present in one or more copies.

Cas9 can refer to a polypeptide with at least or at least about 50%, 60%, 70%, 80%, 90%, 100% sequence identity and/or sequence similarity to a wild type exemplary Cas9 polypeptide (e.g., Cas9 from S. pyogenes). Cas9 can refer to a polypeptide with at most or at most about 50%, 60%, 70%, 80%, 90%, 100% sequence identity and/or sequence similarity to a wild type exemplary Cas9 polypeptide (e.g. , from S. pyogenes). Cas9 can refer to the wild type or a modified form of the Cas9 protein that can comprise an amino acid change such as a deletion, insertion, substitution, variant, mutation, fusion, chimera, or any combination thereof.

A polynucleotide encoding an endonuclease (e.g., a Cas protein such as Cas9) can be codon optimized for expression in particular cells, such as eukaryotic cells. This type of optimization can entail the mutation of foreign-derived (e.g., recombinant) DNA to mimic the codon preferences of the intended host organism or cell while encoding the same protein.

CRISPR enzymes used in the methods can comprise NLSs. The NLS can be located anywhere within the polypeptide chain, e.g., near the N- or C-terminus. For example, the NLS can be within or within about 1, 2, 3, 4, 5, 10, 15, 20, 25, 30, 40, 50 amino acids along a polypeptide chain from the N- or C- terminus. Sometimes the NLS can be within or within about 50 amino acids or more, e.g., 100, 200, 300, 400, 500, 600, 700, 800, 900, or 1000 amino acids from the N- or C-terminus.

An endonuclease can comprise an amino acid sequence having at least or at least about 50%, 60%, 70%, 75%, 80%, 85%, 90%, 95%, 99%, or 100%, amino acid sequence identity to the nuclease domain of a wild type exemplary site-directed polypeptide (e.g., Cas9 from S. *pyogenes*).

While *S. pyogenes* Cas9 (SpCas9), is commonly used as a CRISPR endonuclease for genome engineering, it may not be the best endonuclease for every target excision site. For example, the PAM sequence for SpCas9 (5' NGG 3') is abundant throughout the human genome, but a NGG sequence may not be positioned correctly to target a desired gene for modification. In some cases, a different endonuclease may be used to target certain genomic targets. In some cases, synthetic SpCas9-derived variants with non-NGG PAM sequences may be used. Additionally, other Cas9 orthologues from various species have been identified and these "non-SpCas9s" bind a variety of PAM sequences that could also be useful for the present invention. For example, the relatively large size of SpCas9 (approximately 4kb coding sequence) means that plasmids carrying the SpCas9 cDNA may not be efficiently expressed in a cell. Conversely, the coding sequence for Staphylococcus aureus Cas9 (SaCas9) is approximately 1 kilo base shorter than SpCas9, possibly allowing it to be efficiently expressed in a cell. Similar to SpCas9, the SaCas9 endonuclease is capable of modifying target genes in mammalian cells in vitro and in mice *in vivo.*

Alternatives to *S. pyogenes* Cas9 may include RNA-guided endonucleases from the Cpf 1 family that display cleavage activity in mammalian cells. Unlike Cas9 nucleases, the result of Cpf 1 -mediated DNA cleavage is a double-strand break with a short 3' overhang. Cpfl's staggered cleavage pattern may open up the possibility of directional gene transfer, analogous to traditional restriction enzyme cloning, which may increase the efficiency of gene editing. Like the Cas9 variants and orthologues described above, Cpfl may also expand the number of sites that can be targeted by CRISPR to AT- rich regions or AT-rich genomes that lack the NGG PAM sites favored by SpCas9.

Any functional concentration of Cas protein can be introduced to a cell. For example, 15 micrograms of Cas mRNA can be introduced to a cell. In other cases, a Cas mRNA can be introduced from 0.5 micrograms to 100 micrograms. A Cas mRNA can be introduced from 0.5, 5, 10, 15, 20, 25, 30, 35, 40, 45, 50, 55, 60, 65, 70, 75, 80, 85, 90, 95, or 100 micrograms,

As used herein, the terms "guide RNA (gRNA)" and "single guide RNA (sgRNA)", and their grammatical equivalents can refer to an RNA which can be specific for a target DNA and can form a complex with a Cas protein. A guide RNA can comprise a guide sequence, or spacer sequence, that specifies a target site and guides an RNA/Cas complex to a specified target DNA for cleavage. Site-specific cleavage of a target DNA occurs at locations determined by both 1) base-pairing complementarity between a guide RNA and a target DNA (also called a protospacer) and 2) a short motif in a target DNA referred to as a protospacer adjacent motif (PAM).

A method disclosed herein also can comprise introducing into a cell or embryo at least one guide RNA or nucleic acid, e.g., DNA encoding at least one guide RNA. A guide RNA can interact with a RNA-guided endonuclease to direct the endonuclease to a specific target site, at which site the 5' end of the guide RNA base pairs with a specific protospacer sequence in a chromosomal sequence.

A guide RNA can comprise two RNAs, e.g., CRISPR RNA (crRNA) and transactivating crRNA (tracrRNA). A guide RNA can sometimes comprise a single-guide RNA (sgRNA) formed by fusion of a portion (e.g., a functional portion) of crRNA and tracrRNA. A guide RNA can also be a dual RNA comprising a crRNA and a tracrRNA. A guide RNA can comprise a crRNA and lack a tracrRNA. Furthermore, a crRNA can hybridize with a target DNA or protospacer sequence.

As discussed above, a guide RNA can be an expression product. For example, a DNA that encodes a guide RNA can be a vector comprising a sequence coding for the guide RNA. A guide RNA can be transferred into a cell or organism by transfecting the cell or organism with an isolated guide RNA or plasmid DNA comprising a sequence coding for the guide RNA and a promoter. A guide RNA can also be transferred into a cell or organism in other way, such as using virus-mediated gene delivery.

A guide RNA can be isolated. For example, a guide RNA can be transfected in the form of an isolated RNA into a cell or organism. A guide RNA can be prepared by in vitro transcription using any in vitro transcription system. A guide RNA can be transferred to a cell in the form of isolated RNA rather than in the form of plasmid comprising encoding sequence for a guide RNA.

A guide RNA can comprise a DNA-targeting segment and a protein binding segment. A DNA- targeting segment (or DNA-targeting sequence, or spacer sequence) comprises a nucleotide sequence that can be complementary to a specific sequence within a target DNA (e.g., a protospacer). A protein-binding segment (or protein-binding sequence) can interact with a site-directed modifying polypeptide, e.g. an RNA -guided endonuclease such as a Cas protein. By "segment" it is meant a segment/section/region of a molecule, e.g., a contiguous stretch of nucleotides in an RNA. A segment can also mean a region/section of a complex such that a segment may comprise regions of more than one molecule. For example, in some cases a protein-binding segment of a DNA-targeting RNA is one RNA molecule and the protein-binding segment therefore comprises a region of that RNA molecule. In other cases, the protein-binding segment of a DNA-targeting RNA comprises two separate molecules that are hybridized along a region of complementarity.

A guide RNA can comprise two separate RNA molecules or a single RNA molecule. An exemplary single molecule guide RNA comprises both a DNA-targeting segment and a protein-binding segment.

An exemplary two-molecule DNA-targeting RNA can comprise a crRNA -like ("CRISPR RNA" or "targeter-RNA" or "crRNA" or "crRNA repeat") molecule and a corresponding tracrRNA -like ("transacting CRISPR RNA" or "activator-RNA" or "tracrRNA") molecule. A first RNA molecule can be a crRNA -like molecule (targeter-RNA), that can comprise a DNA-targeting segment (e.g., spacer) and a stretch of nucleotides that can form one half of a double-stranded RNA (dsRNA) duplex comprising the protein-binding segment of a guide RNA. A second RNA molecule can be a corresponding tracrRNA -like molecule (activator-RNA) that can comprise a stretch of nucleotides that can form the other half of a dsRNA duplex of a protein-binding segment of a guide RNA. In other words, a stretch of nucleotides of a crRNA -like molecule can be complementary to and can hybridize with a stretch of nucleotides of a tracrRNA -like molecule to form a dsRNA duplex of a protein-binding domain of a guide RNA. As such, each crRNA-like molecule can be said to have a corresponding tracrRNA-like molecule. A crRNA-like molecule additionally can provide a single stranded DNA-targeting segment, or spacer sequence. Thus, a crRNA-like and a tracrRNA-like molecule (as a corresponding pair) can hybridize to form a guide RNA. A subject two-molecule guide RNA can comprise any corresponding crRNA and tracrRNA pair.

A DNA-targeting segment or spacer sequence of a guide RNA can be complementary to sequence at a target site in a chromosomal sequence, e.g., protospacer sequence) such that the DNA-targeting segment of the guide RNA can base pair with the target site or protospacer. In some cases, a DNA- targeting segment of a guide RNA can comprise from or from about 10 nucleotides to from or from about 25 nucleotides or more. For example, a region of base pairing between a first region of a guide RNA and a target site in a chromosomal sequence can be or can be about 10, 11, 12, 13, 14, 15, 16, 17, 18, 19, 20, 22, 23, 24, 25, or more than 25 nucleotides in length. Sometimes, a first region of a guide RNA can be or can be about 19, 20, or 21 nucleotides in length.

A guide RNA can target a nucleic acid sequence of or of about 20 nucleotides. A target nucleic acid can be less than or less than about 20 nucleotides. A target nucleic acid can be at least or at least about 5, 10, 15, 16, 17, 18, 19, 20, 21, 22, 23, 24, 25, 30 or more nucleotides. A target nucleic acid can be at most or at most about 5, 10, 15, 16, 17, 18, 19, 20, 21, 22, 23, 24, 25, 30 or more nucleotides. A target nucleic acid sequence can be or can be about 20 bases immediately 5' of the first nucleotide of the PAM. A guide RNA can target the nucleic acid sequence.

A guide nucleic acid, for example, a guide RNA, can refer to a nucleic acid that can hybridize to another nucleic acid, for example, the target nucleic acid or protospacer in a genome of a cell. A guide nucleic acid can be RNA. A guide nucleic acid can be DNA. The guide nucleic acid can be programmed or designed to bind to a sequence of nucleic acid site-specifically. A guide nucleic acid can comprise a polynucleotide chain and can be called a single guide nucleic acid. A guide nucleic acid can comprise two polynucleotide chains and can be called a double guide nucleic acid.

A guide nucleic acid can comprise one or more modifications to provide a nucleic acid with a new or enhanced feature. A guide nucleic acid can comprise a nucleic acid affinity tag. A guide nucleic acid can comprise synthetic nucleotide, synthetic nucleotide analog, nucleotide derivatives, and/or modified nucleotides.

A guide nucleic acid can comprise a nucleotide sequence (e.g. , a spacer), for example, at or near the 5' end or 3' end, that can hybridize to a sequence in a target nucleic acid (e.g. , a protospacer). A spacer of a guide nucleic acid can interact with a target nucleic acid in a sequence-specific manner via hybridization (i.e., base pairing). A spacer sequence can hybridize to a target nucleic acid that is located 5' or 3' of a protospacer adjacent motif (PAM). The length of a spacer sequence can be at least or at least about 5, 10, 15, 16, 17, 18, 19, 20, 21, 22, 23, 24, 25, 30 or more nucleotides. The length of a spacer sequence can be at most or at most about 5, 10, 15, 16, 17, 18, 19, 20, 21, 22, 23, 24, 25, 30 or more nucleotides. [00337] A guide RNA can also comprises a dsRNA duplex region that forms a secondary structure. For example, a secondary structure formed by a guide RNA can comprise a stem (or hairpin) and a loop. A length of a loop and a stem can vary. For example, a loop can range from about 3 to about 10 nucleotides in length, and a stem can range from about 6 to about 20 base pairs in length. A stem can comprise one or more bulges of 1 to about 10 nucleotides. The overall length of a second region can range from about 16 to about 60 nucleotides in length. For example, a loop can be or can be about 4 nucleotides in length and a stem can be or can be about 12 base pairs. A dsRNA duplex region can comprise a protein-binding segment that can form a complex with an RNA -binding protein, such as a RNA-guided endonuclease, e.g. Cas protein.

A guide RNA can also comprise a tail region at the 5' or 3' end that can be essentially single-stranded.

For example, a tail region is sometimes not complementarity to any chromosomal sequence in a cell of interest and is sometimes not complementarity to the rest of a guide RNA. Further, the length of a tail region can vary. A tail region can be more than or more than about 4 nucleotides in length. For example, the length of a tail region can range from or from about 5 to from or from about 60 nucleotides in length.

A guide RNA can be introduced into a cell or embryo as an RNA molecule. For example, a RNA molecule can be transcribed in vitro and/or can be chemically synthesized. A guide RNA can then be introduced into a cell or embryo as an RNA molecule. A guide RNA can also be introduced into a cell or embryo in the form of a non-RNA nucleic acid molecule, e.g., DNA molecule. For example, a DNA encoding a guide RNA can be operably linked to promoter control sequence for expression of the guide RNA in a cell or embryo of interest. A RNA coding sequence can be operably linked to a promoter sequence that is recognized by RNA polymerase III (Pol III).

A DNA molecule encoding a guide RNA can also be linear. A DNA molecule encoding a guide RNA can also be circular.

A DNA sequence encoding a guide RNA can also be part of a vector. Some examples of vectors can include plasmid vectors, phagemids, cosmids, artificial/mini-chromosomes, trans-posons, and viral vectors. For example, a DNA encoding a RNA-guided endonuclease is present in a plasmid vector. Other non-limiting examples of suitable plasmid vectors include pUC, pBR322, pET, pBluescript, and variants thereof. Further, a vector can comprise additional expression control sequences (e.g., enhancer sequences, Kozak sequences, polyadenylation sequences, transcriptional termination sequences, etc.), selectable marker sequences (e.g., antibiotic resistance genes), origins of replication, and the like.

When both a RNA-guided endonuclease and a guide RNA are introduced into a cell as DNA molecules, each can be part of a separate molecule (e.g., one vector containing fusion protein coding sequence and a second vector containing guide RNA coding sequence) or both can be part of a same molecule (e.g., one vector containing coding (and regulatory) sequence for both a fusion protein and a guide RNA). A Cas protein, such as a Cas9 protein or any derivative thereof, can be pre-complexed with a guide RNA to form a ribonucleoprotein (RNP) complex. The RNP complex can be introduced into primary immune cells. Introduction of the RNP complex can be timed. The cell can be synchronized with other cells at Gl, S, and/or M phases of the cell cycle. The RNP complex can be delivered at a cell phase such that HDR is enhanced. The RNP complex can facilitate homology directed repair.

A guide RNA can also be modified. The modifications can comprise chemical alterations, synthetic modifications, nucleotide additions, and/or nucleotide subtractions. The modifications can also enhance CRISPR genome engineering. A modification can alter chirality of a gRNA. In some cases, chirality may be uniform or stereopure after a modification. A guide RNA can be synthesized. The synthesized guide RNA can enhance CRISPR genome engineering. A guide RNA can also be truncated. Truncation can be used to reduce undesired off-target mutagenesis. The truncation can comprise any number of nucleotide deletions. For example, the truncation can comprise 1, 2, 3, 4, 5, 10, 15, 20, 25, 30, 40, 50 or more nucleotides. A guide RNA can comprise a region of target complementarity of any length. For example, a region of target complementarity can be less than 20 nucleotides in length. A region of target complementarity can be more than 20 nucleotides in length.

In some cases, a dual nickase approach may be used to introduce a double stranded break, which is preferable in context of the present invention to avoid off-target effects. Cas proteins can be mutated at known amino acids within either nuclease domains, thereby deleting activity of one nuclease domain and generating a nickase Cas protein capable of generating a single strand break. A nickase along with two distinct guide RNAs targeting opposite strands may be utilized to generate a DSB within a target site (often referred to as a "double nick" or "dual nickase" CRISPR system). This approach may dramatically increase target specificity, since it is unlikely that two off- target nicks will be generated within close enough proximity to cause a DSB. The examples of the present application describe the use of a double nickase CRISPR approach to knowck out both IKK1 and IKK2. The term "nickase" as used herein refers to an endonuclease which cleaves only a single strand of a DNA duplex. The term "Cas9 nickase" refers to a nickase derived from a Cas9 protein, typically by inactivating one nuclease domain of Cas9 protein.

In some cases, a GUIDE-Seq analysis can be performed to determine the specificity of engineered guide RNAs. The general mechanism and protocol of GUIDE-Seq profiling of off-target cleavage by CRISPR system nucleases is discussed in Tsai, S. et al, "GUIDE-Seq enables genome-wide profiling of off-target cleavage by CRISPR system nucleases," Nature, 33: 187-197 (2015).

A gRNA can be introduced at any functional concentration. For example, a gRNA can be introduced to a cell at 10 micrograms. In other cases, a gRNA can be introduced from 0.5 micrograms to 100 micrograms. A gRNA can be introduced from 0.5, 5, 10, 15, 20, 25, 30, 35, 40, 45, 50, 55, 60, 65, 70, 75, 80, 85, 90, 95, or 100 micrograms.

In some cases, a method can comprise an endonuclease selected from the group consisting of Casi, CasiB, Cas2, Cas3, Cas4, Cas5, Cas6, Cas7, Cas8, Cas9, CasiO, Csy1, Csy2, Csy3, Cse1, Cse2, Csc1, Csc2, Csa5, Csn2, Csm2, Csm3, Csm4, Csm5, Csm6, Cmr1, Cmr3, Cmr4, Cmr5, Cmr6, Csb1, Csb2, Csb3, Csxl7, Csxl4, Csx1O, Csx16, CsaX, Csx3, Csx1, Csx1S, Csf1, Csf2, CsO, Csf4, Cpf1, c2c1, c2c3, Cas9HiFi, homologues thereof or modified versions thereof. A Cas protein can be Cas9.

In some cases, a method can further comprise at least one guide RNA (gRNA). A gRNA can comprise at least one modification, for example by introducing at least one guide RNA (gRNA) comprising at least one modification; and introducing at least one endonuclease; wherein the gRNA comprises at least one sequence complementary to at least one endogenous genome. In some cases, a modification is on a 5' end, a 3' end, from a 5' end to a 3' end, a single base modification, a 2'-ribose modification, or any combination thereof. A modification can be selected from a group consisting of base substitutions, insertions, deletions, chemical modifications, physical modifications, stabilization, purification, and any combination thereof.

In context of the invention it is preferred that at least IKK1 and IKK2 are knocked out using any system available to the skilled artisan. Therefore, approaches are preferred wherein at least two, preferably three, more preferably four gRNA are used for targeting each of IKK1 and IKK2, such as gRNA comprising a nucleotide sequence complementary to the sense or antisense strand of the IKK1 and/or IKK2 genes. Preferably at least one pair of gRNAs is used for targeting each of IKK1 and IKK2, and wherein the two gRNA of each pair of gRNA(s) target a sequence on opposite strands of the target gene. In some embodiments the target sequences on opposite strands are not more than 15, preferably not more than 10, nucleotides apart from each other.

Most preferably at least two pairs of gRNA molecules are used for targeting each of IKK1 and IKK2 according to the aforementioned double nickase CRISPR approach.

In context of the invention as described above, the use of gene editing requires the introduction of a gene editing nuclease into the cell, for example by introducing into the cell an expressible DNA or RNA construct encoding for the gene editing nuclease, or introducing into the cell a gene editing nuclease protein. The respective nucleases usable for the invention re described herein above and preferably are Cpf1 or Cas9, or an optimized nuclease such as Cas9-HF1 or eSpCas9, but preferably is a nickase, such as Cas9 D10A and Cas9 H840A. Many nucleases usable in gene editing are known to date and the invention shall not be restricted to any particular enzyme sequence.

Gene editing for the targeted knock out IKK1 and IKK2 preferably involves the use of a gRNA selected from SEQ ID NO: 1 to 4 (for IKK1) and/or SEQ ID NO: 5 to 8 (forIKK2).

The gene editing approach of the invention in some embodiments preferably introduces a deletion into the genes for IKK1 and IKK2. Such a deletion preferably results in a complete loss of function of IKK1 and IKK2 in the cell associated with the proliferative disorder.

Conventional viral and non-viral based gene transfer methods can be used to introduce nucleic acids encoding engineered CRISPR, TALEN, transposon-based, ZEN, meganuclease, or Mega-TAL molecules and/or transgenes in cells (e.g., mammalian cells) and target tissues. Such methods can also be used to administer nucleic acids encoding CRISPR, TALEN, transposon-based, ZEN, meganuclease, or Mega-TAL molecules and/or transgenes to cells *in vitro.* In some examples, nucleic acids encoding CRISPR, TALEN, transposon-based, ZEN, meganuclease, or Mega-TAL molecules and/or transgenes can be administered for in vivo or ex vivo immunotherapy uses. Non-viral vector delivery systems can include DNA plasmids, naked nucleic acid, and nucleic acid complexed with a delivery vehicle such as a liposome or poloxamer. Viral vector delivery systems can include DNA and RNA viruses, which have either episomal or integrated genomes after delivery to the cell.

Alternatively, naked DNA or mRNA can be administered. Administration is by any of the routes normally used for introducing a molecule into ultimate contact with blood or tissue cells including, but not limited to, injection, infusion, topical application and electro-poration. More than one route can be used to administer a particular composition. Pharmaceutically acceptable carriers are determined in part by the particular composition being administered, as well as by the particular method used to administer the composition.

A gRNA, or gRNA expression construct, or said gene editing nuclease, or gene editing nuclease expressing construct, is in preferred embodiments encapsulated in a viral particle or a nanoparticle for cellular delivery. Preferred systems for delivery according to the invention include a lentivirus, adenovirus, or adeno-associated virus (AAV).

In some preferred aspects of the invention said viral particle or said nanoparticle comprises a targeting moiety for specifically targeting the particle to the cell associated with the disease. Such a targeting moiety is preferably a molecule that specifically binds to a cell associated with the proliferative disease. Specific examples of targeting moieties of for example tumor cells are well known in the art and include antibodies or antibody derived molecules or T cell receptor constructs which are specific and selective for binding tumor cell expressed TAA or TSA.

In some embodiments the method of treatment according to the invention comprises a step of activating TNF mediated signaling in the cell associated with the disease. Preferably, activating Tumor Necrosis Factor (TNF) mediated signaling in the cell associated with the disease comprises TNF receptor (TNFR)-activation, for example by using TNF α, a TNF αanalog, or a TNFR agonist. TNFR signaling may promote both cellular proliferation or the activation of the apoptotic pathway leading to programmed cell death. Inactivating IKK1 and/or IKK2 according to the invention however results in the induction of the apoptotic pathway via TNFR.

In certain embodiments of the various aspects of the invention, cells involved with the proliferative disorder are those exposed to an appropriate triggering or activating molecule, such as TNF, a variant of TNF and or an agonist of TNFR2- or TNFR1-signalling (preferably, an agonist of TNFR1-signalling), in particular are exposed to an effective amount of a molecule that acts agonistic to TNF. TNF in context of the invention is preferably a human TNF. In certain of such embodiments, the TNF is recombinant human TNF (rHuTNF). However, in other embodiments the TNF is endogenous TNF, such as that is produced by or otherwise present in the subject (eg the human patient).

It is known from studies that plasma TNF levels are elevated in numerous types of cancers, and that for example, the upper normal limit of total TNF in healthy subjects is 1.8 pg/mL, as measured using; a Quantikine human TNF-alpha Immunoassay PDTAooC, including in ovarian cancer (Dobrzycka et al 2009, Eur Cytokine Netw 20:131). In other cancers and assays (eg, TNF-alpha-ELISA Kit, DIAsource), the TNF plasma levels of oesophageal cancer patients and the control group were 12.35 ± 9.69 and 4.62 ± 3.06 pg/ mL, respectively (Ay-din et al 2012, Turk J Med Sci 42:762). Accordingly, in other embodiments the cells associated with the proliferative disorder are (for example a tumour is) one present in a subject having a plasma concentration of TNF greater than about 1.5, 2.5 or 4 pg/mL, such as greater than about 5 pg/mL, and in particular greater than about 10 pg/mL (for example, as measured by a Quantikine human TNF-alpha Immunoassay PDTAooC or a TNF-alpha-ELISA Kit, DIAsource).

Accordingly, in one particular embodiment, the subject involved in the treatment methods of the invention may have a plasma concentration of TNF greater than about 2 pg/mL (eg, the cells involved with the proliferative disorder are one present in a subject having a plasma concentration of TNF greater than about 2 pg/mL).

Also, in such embodiments when the proliferative disorder is a tumour (eg a solid tumour), then the solid tumour (eg, within the subject) may have an intratumoural concentration of TNF greater than 0.5 pg/mL.

The invention hereby provides alternative combination treatment regimens based on the surprising finding of the inventors that a dual inactivation of IKK1 and IKK2 can increase the sensitivity of a cell towards the cytotoxic effects of TNF. Accordingly, in a fourth aspect, and as may be further described, defined, claimed or otherwise disclosed herein, the invention relates to a method for the treatment of a proliferative disorder in a subject, the method comprising exposing (eg contacting) cells involved with the proliferative disorder in the subject to: (i) TNF, a TNF variant and/or an agonist of TNFR2- or TNFR1-signalling; and exposing (eg contacting) the cells involved with the proliferative disorder in the subject to (ii) a compound or composition of compounds having antagonistic activity towards IKK1 and(preferred)/or IKK2.

In such embodiments, the antagonists of IKK1 and/or IKK2 and the TNF, TNF variant or TNFR1 or TNFR2 agonist can be exposed to (for example administered in) an effective amount (or dose), including in formulations or administrative routes as described elsewhere herein. In particular are envisioned embodiments where the TNF, TNF variant or TNFR1 or TNFR2 agonist is encapsulated as a liposomal or other nanoparticle formulation.

When the TNF, TNF variant or TNFR1 or TNFR2 agonist is exposed/administered and the IKK antagonist is exposed/administered, then such combination treatment regimen may comprise embodiments where such exposures/administrations are concomitant. In alternative embodiments such exposures/administrations may be sequential.

The TNF or the TNF variant or TNFR1 or TNFR2 agonist may be administered via conventional routes, such as s.c., i.v. or i.m., and on certain embodiments may be administered intratumourally or by isolated limb perfusion (ILP), such as isolated hepatic perfusion (IHP); and/or may be so administered (in particular, rHuTNF may be so administered) at a dose of between about 5 and 500 ug/m2/day. For example, TNF may be administered between about 25 and 250 ug/m2/day, such as between about 50 and 150 ug/m2/day or between about 75 and 100 ug/m2/day; or wherein TNF is administered up to a MTD of about 50 and 75 ug/m2/day when administered s.c. or up to a MTD of about 150 and 200 ug/m2/day when administered i.v. or i.m. Accordingly, in particular of such embodiments, TNF can be administered to the subject at a dose of between about 5 and 500 ug/m2/day, in particular between about 20 and 200 ug/m2/day.

In particular embodiments a variant of TNF, such as a TNF variant having higher antitumour activity and lower systemic toxicity that rHuTNF may be exposed/administered. For example, the TNF variant may be one selected from the group consisting of: (i) a - K90R variant of TNF; (ii) a tumour-homing peptide conjugated to TNF; and (iii) a TNF-antibody conjugate. In other embodiments a TNFR1 or TNFR2 agonist, such as the anti-TNFR1 monoclonal antibody htr-9 (Ferrero et al 2001, Am J Physiol Cell Physiol 281:C1173) may be exposed/administered, and in other embodiments lymphotoxin-alpha (Etemadi et al 2013, FEBS J 280:5283) or a variant thereof may be exposed/administered.

In certain of such embodiments, the agent is a virus, in particular one that has been engineered to produce a triggering molecule being TNF, a TNF variant or the TNFR1 or TNFR2 agonist (especially, a virus engineered to produce human TNF). Further of such embodiments include those where such virus preferentially infects the cell(s) involved with the proliferative disorder (eg tumour cells) and/or preferentially produces the triggering molecule in the context of (eg when it infects) such cells. As will now be apparent, the administration of such a virus can lead to the exposure of the cell(s) involved with the proliferative disorder to such triggering molecule, and in particular to an effective amount of such a triggering molecule such as TNF.

In another aspect the disclosure provides a composition for use in a method for treating a proliferative disorder in a subject, for example as described herein above, the composition comprising an inhibitor/antagonist of IKK1 and an inhibitor/antagonist of IKK2. Such a composition may in some preferred aspects and embodiments be formulated as a pharmaceutical composition, which are described herein in the following:

The pharmaceutical composition of the invention is formulated to be compatible with its intended route of administration. Examples of routes of administration include parenteral, e.g., intrathecal, intra-arterial, intravenous, intradermal, subcutaneous, oral, transdermal (topical), intracerebroventricular, intraparenchymal, intratumoral and transmucosal administration. Generally a pharmaceutical composition of the invention shall comprise one or more compounds for antagonizing IKK1 and/or IKK2, preferably for IKK1 and IKK2, and at least one pharmaceutically acceptable carrier and/or excipient. The pharmaceutical composition as described herein are particularly useful for use in the herein described methods for treating a proliferative disorder.

The term "intrathecal," as used herein, means introduced into or occurring in the space under the arachnoid membrane which covers the brain and spinal cord. The term "intracerebroventricular" refers to administration of a composition into the ventricular system of the brain, e.g., via injection, infusion, or implantation (for example, into a ventricle of the brain). As used herein, the term "intraparenchymal" can refer to an administration directly to brain tissue. In other instances, intraparenchymal administration may be directed to any brain region where delivery of one or more compounds of the invention is effective to mitigate or prevent one or more of disorders as described herein. Forms of administration directly to brain tissue is on some embodiments preferred.

Solutions or suspensions used for parenteral, intradermal, or subcutaneous application can include the following components: a sterile diluent such as water for injection, saline solution, fixed oils, polyethylene glycols, glycerine; propylene glycol or other synthetic solvents; anti-bacterial agents such as benzyl alcohol or methyl parabens; antioxidants such as ascorbic acid or sodium bisulfate; chelating agents such as ethylenediaminetetraacetic acid; buffers such as acetates, citrates or phosphates and agents for the adjustment of tonicity such as sodium chloride or dextrose. pH can be adjusted with acids or bases, such as hydrochloric acid or sodium hydroxide. The parenteral preparation can be enclosed in ampoules, disposable syringes or multiple dose vials made of glass or plastic.

Pharmaceutical compositions suitable for injectable use include sterile aqueous solutions (where water soluble) or dispersions and sterile powders for the extemporaneous preparation of sterile injectable solutions or dispersion. For intravenous administration, suitable carriers include physiological saline, bacteriostatic water, Cremophor EL™ (BASF, Parsippany, N.J.) or phosphate buffered saline (PBS). In all cases, the injectable composition should be sterile and should be fluid to the extent that easy syringability exists. It must be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol, and liquid polyetheylene glycol, and the like), and suitable mixtures thereof. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, it will be preferable to include isotonic agents, for example, sugars, polyalcohols such as mannitol, sorbitol, and sodium chloride in the composition. Prolonged absorption of the injectable compositions can be brought about by including in the composition an agent which delays absorption, for example, aluminum monostearate and gelatin.

Sterile injectable solutions can be prepared by incorporating the active compound (e.g., a sulfotransferase inhibitor) in the required amount in an appropriate solvent with one or a combination of ingredients enumerated above, as required, followed by filtered sterilization. Generally, dispersions are prepared by incorporating the active compound into a sterile vehicle which contains a basic dispersion medium and the required other ingredients from those enumerated above. In the case of sterile powders for the preparation of sterile injectable solutions, the preferred methods of preparation are vacuum drying and freezedrying which yields a powder of the active ingredient plus any additional desired ingredient from a previously sterile-filtered solution thereof.

Oral compositions generally include an inert diluent or an edible carrier. They can be enclosed in gelatin capsules or compressed into tablets. For the purpose of oral therapeutic administration, the active compound can be incorporated with excipients and used in the form of tablets, troches, or capsules. Oral compositions can also be prepared using a fluid carrier for use as a mouthwash, wherein the compound in the fluid carrier is applied orally and swished and expectorated or swallowed. Pharmaceutically compatible binding agents, and/or adjuvant materials can be included as part of the composition. The tablets, pills, capsules, troches and the like can contain any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate or Stertes; a glidant such as colloidal silicon dioxide; a sweetening agent such as sucrose or saccharin; or a flavoring agent such as peppermint, methyl salicylate, or orange flavoring.

For administration by inhalation, the compounds are delivered in the form of an aerosol spray from pressured container or dispenser which contains a suitable propellant, e.g., a gas such as carbon dioxide, or a nebulizer.

Systemic administration can also be by transmucosal or transdermal means. For transmucosal or transdermal administration, penetrants appropriate to the barrier to be permeated are used in the formulation. Such penetrants are generally known in the art, and include, for example, for transmucosal administration, detergents, bile salts, and fusidic acid derivatives. Transmucosal administration can be accomplished through the use of nasal sprays or suppositories. For transdermal administration, the pharmaceutical compositions are formulated into ointments, salves, gels, or creams as generally known in the art.

In certain embodiments, the pharmaceutical composition is formulated for sustained or controlled release of the active ingredient. Biodegradable, biocompatible polymers can be used, such as ethylene vinyl acetate, polyanhydrides, polyglycolic acid, collagen, polyorthoesters, and polylactic acid. Methods for preparation of such formulations will be apparent to those skilled in the art. The materials can also be obtained commercially from e.g. Alza Corporation and Nova Pharmaceuticals, Inc. Liposomal suspensions (including liposomes targeted to infected cells with monoclonal antibodies to viral antigens) can also be used as pharmaceutically acceptable carriers. These can be prepared according to methods known to those skilled in the art.

It is especially advantageous to formulate oral or parenteral compositions in dosage unit form for ease of administration and uniformity of dosage. Dosage unit form as used herein includes physically discrete units suited as unitary dosages for the subject to be treated; each unit containing a predetermined quantity of active compound calculated to produce the desired therapeutic effect in association with the required pharmaceutical carrier. The specification for the dosage unit forms of the invention are dictated by and directly dependent on the unique characteristics of the active compound and the particular therapeutic effect to be achieved, and the limitations inherent in the art of compounding such an active compound for the treatment of individuals.

Toxicity and therapeutic efficacy of such compounds can be determined by standard pharmaceutical procedures in cell cultures or experimental animals, e.g., for determining the LD50 (the dose lethal to 50% of the population) and the ED50 (the dose therapeutically effective in 50% of the population). The dose ratio between toxic and therapeutic effects is the therapeutic index and it can be expressed as the ratio LD50/ED50. Compounds which exhibit large therapeutic indices are preferred. While compounds that exhibit toxic side effects may be used, care should be taken to design a delivery system that targets such compounds to the site of affected tissue in order to minimize potential damage to uninfected cells and, thereby, reduce side effects.

The data obtained from the cell culture assays and animal studies can be used in formulating a range of dosage for use in humans. The dosage of such compounds lies preferably within a range of circulating concentrations that include the ED50 with little or no toxicity. The dosage may vary within this range depending upon the dosage form employed and the route of ad-ministration utilized. For any compound used in the method of the invention, the therapeutically effective dose can be estimated initially from cell culture assays. A dose may be formulated in animal models to achieve a circulating plasma concentration range that includes the IC50 (i.e., the concentration of the test compound which achieves a half-maximal inhibition of symptoms) as determined in cell culture. Such information can be used to more accurately determine useful doses in humans. The pharmaceutical compositions can be included in a container, pack, or dispenser together with instructions for administration.

The present invention will now be further described in the following examples with reference to the accompanying figures and sequences, nevertheless, without being limited thereto. For the purposes of the present invention, all references as cited herein are incorporated by reference in their entireties. In the Figures:
- **Figure 1:**: Target designs for the CRISPR/Cas9 system. Gene sequences were taken from Ensembl Genome Browser (ensemble.org), sgRNAs designed with the Target Online Predictor from the University of Heidelberg (crispr.cos.uniheidelberg.de). The created double-strand-breaks were created around intron-exon-borders. A: IKK2; B: IKK1
- **Figure 2**:: Immunoblot Scheme

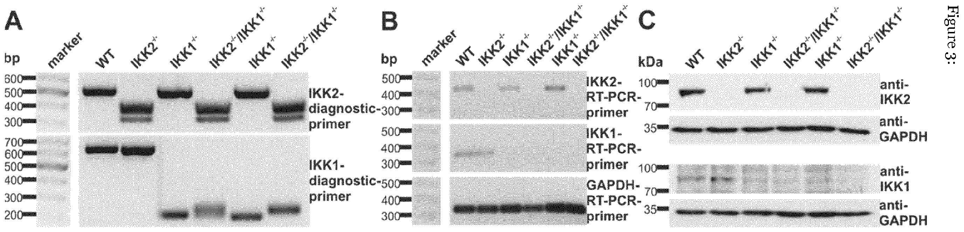

- **Figure 3**:: Validation of knockout-clones. A: Genomic PCR: On the upper the results of the genomic PCR with IKK2-deagnostic-primers is shown. Wildtype bands run at about 500 bp, the selected knockout-clone shows two bands, one at around 400 bp, the other slightly above 300 bp. On the lower with IKK1-diagnostic-primers, wildtype bands run slightly above 600 bp, selected knockout-clones at 200 bp respective slightly above. B: RT-PCR: On the upper the IKK2-RT-PCR-primer-pair was used, in the middle the IKK1-RT-PCR-primer-pair and on the lower GAPDH-RT-PCR-primers. The latter serving as a loading control. Absence of bands shows absence of intact mRNA. C: Western-blot: On the upper an anti-IKK2-antibody was used, bands at around 87 kDa show presence of IKK2. On the lower an anti IKK1-antibody was used, the presence of protein shows in bands at around 85 kDa. In both cases a GAPDH loading control was performed and is shown below each
- **Figure 4**:: Cells after TNFα-stimulation and control. From the top: wildtype, IKK2-knockout, IKK1-knockout, double-knockout. On the left control, on the right cells stimulated with TNFα at 20 ng/ml.
- **Figure 5**:: Results of propidium iodide (PI) flow cytometric assay. Cell counts plotted dependent on size of nucleus. From the top: wildtype, IKK2-knockout, IKK1-knockout, double-knockout. From left to right: control, TNFα-stimulated, overlay.

### EXAMPLES

### Material and Methods

### Design of CRISPR Deletions

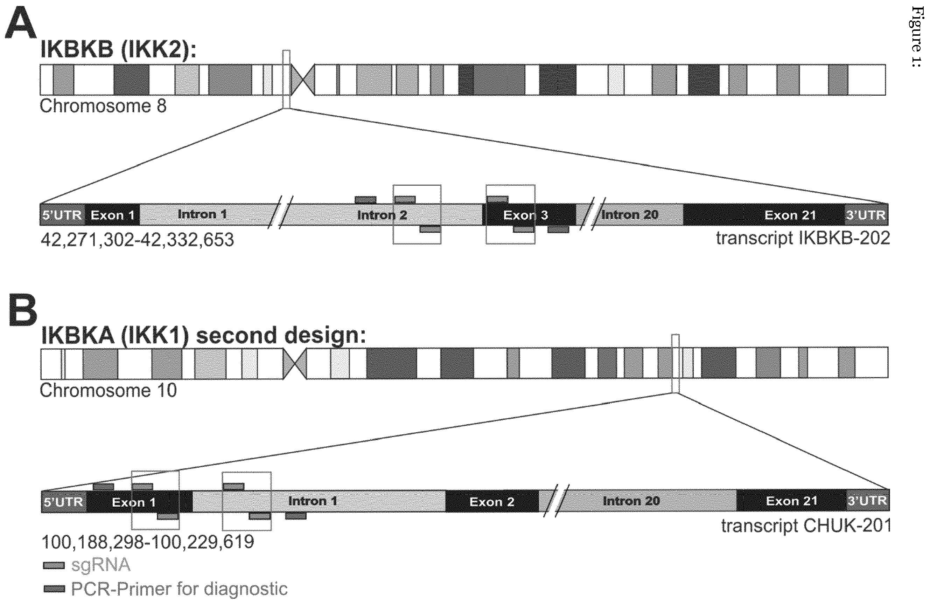

The sgRNA designs were created using the gene sequences from ensemble.org and the CCTop-CRISPR/Cas9 target online predictor (crispr.cos.uni-heidelberg.de). Using the CRISPR/Cas9n-system with nickase activity, 4 sgRNAs are needed to create two double strand breaks {Ran, 2013 #3}. The parameters used to create the designs were oligonucleotide lengths of 20 bp, PAM motif "NGG" (necessary for use of system derived of *Streptococcus pyogenes* {Jinek, 2012 #2}), overhangs of U6-promotor, no more than 10 bp distance between oligos of one nicking pair, a predicted deletion of 700 bp or less and deletion of an intron-exon border. The sgRNAs were chosen in account of having the least potential off-targets possible. The developed designs are shown in Fig. 1.

For annealing of forward and reverse sgRNAs, 1 µl (100 mM) of each were mixed with 6 µl ddH₂O, 1 µl 10x T4 ligation buffer (New England Biolabs, Frankfurt a. M., Germany) and 1 µl of T4 Polynucleotide kinase (PNK) (New England Biolabs). A negative control used ddH₂O instead of oligos. The samples were incubated at 37 °C for 30 min, followed by 95 °C for 5 min before the temperature was ramped down at 5 °C per min until 25 °C were reached.

The annealed oligos were diluted 1:200 and 2 µl were mixed with 11.5 µl ddH₂O, 2µl 10x T4 ligation buffer (New England Biolabs), 1 µl dithiothreitol (DTT) (10 mM) (Roth, Karlsruhe, Germany), 1 µl adenosine triphosphate (ATP) (10 mM) (New England Biolabs), 1 µl BbsI (New England Bioloabs), 1 µl vector (pSpCas9n(BB)-2A-Puro (PX462) V2.0, 100 ng/µl, Plasmid #62987, (addgene, Cambridge, UK)) and 0.5 µl T4 ligase (New England Biolabs). A negative control contained ddH₂O instead of annealed oligos. The samples were incubated 6 times at 37 °C for 5 min followed by 21 °C, for 5 min.

Competent *E. coli* DH5α (Leibniz Institute DSMZ-German Collection of Microorganisms and Cell Cultures, Braunschweig, Germany/competence lab made) were transformed by mixing 50 µl bacteria with the ligation solutions and incubating them 15 min on ice followed by 50 s at 42 °C and additional 10 min on ice. 1 ml SOC-medium (lab-made) was added and the samples were shaken at 37 °C for 30 min. They were centrifuged for 2 min at 6000 rpm, 800 µl supernatant were removed, the pellets resuspended in the remaining supernatant, seeded on LB-Amp (50 µg/ml) plates and incubated at 37 °C overnight.

Colony PCR was used to screen for plasmids with insert, for which colonies were picked, put in 50 µl ddH₂O, heated for 3 min at 95 °C, cooled on ice for 5 min and centrifuged for 10 min at 13000 rpm. The supernatant served as DNA-template and a Colony PCR was performed following the scheme in Tab. 1. A negative control used ddH₂O instead of DNA-template. The samples were analysed using gel electrophoresis on 2 % agarose gel with TRIS-acetate-EDTA (TAE)-buffer (lab-made) and Ethidium bromide (EtBr) (Roth).

**Table 1: Reaction scheme Colony PCR. Negative-control using H₂O instead of template.**

| Colony PCR | |
|---|---|
| Component | Quantity [µl] |
| ddH₂O | 21.75 |
| 5x GoTaq Buffer (Promega, Madison, USA) | 10 |
| Deoxynucleoside triphosphates (dNTPs) (10 Mm) (New England Biolabs) | 1 |
| GoTaq polymerase (Promega) | 0.25 |
| Forward primer (Y828) 10 µM | 1 |
| Reverse primer (one of the sgRNAs) 10 µM | 1 |
| DNA-template | 15 |

**Table 2: Thermocycler program for Colony PCR.**

| Thermocycler program Colony PCR | | |
|---|---|---|
| Repetitions | Time [s] | Temperature [°C] |
| 1X | 120 | 95 |
| 35X | 30 | 95 |
| | 30 | 60 |
| | 30 | 72 |
| 1X | 300 | 72 |

Plasmid DNA of positive colonies was isolated for sequencing. Thus 1.5 ml of an overnight culture were centrifuged for 2 min at 13000 rpm and the supernatant was removed before the pellet was resuspended in 200 µl TELT-buffer (lab-made) supplemented with 20 µl lysozyme (10 mg/ml) (AppliChem, Darmstadt, Germany), incubated for 3 min at 95 °C and 5 min on ice. After 15 min centrifugation at 13000 rpm and 4 °C, the pellet was removed, 100 µl isopropanol (chemical storage, University of Bielefeld, Germany) were added to the supernatant and mixed by inversion. To precipitate the samples, they were incubated at room temperature for 5 min and afterwards centrifuged for 15 min at 13000 rpm and 4 °C before the supernatant was removed. The pellet was washed with 200 µl 70 % ethanol (chemical storage), mixed by inversion and centrifuged for 5 min at 13000 rpm and 4 °C before the supernatant was removed again and the pellet dried for 5 min at room temperature. Lastly, the pellet was resuspended in 30 µl Tris(hydroxymethyl)-aminomethan (TRIS) (10 mM) (pH 8.5) (Roth), incubated at 30 °C for 15 min and send to sequencing (Sequencing Core Facility, CeBiTec, Bielefeld, Germany) with Y828 primer.

The sequencing data was aligned to the theoretical constructs using Serial Cloner (version 2.6, open source (serialbasics.free.fr/Serial_Cloner.html)) and clones containing the correct sequence of the insert were chosen for plasmid midi-prep. The plasmids of 200 ml overnight culture in LB-Amp were purified using NucleoBond Xtra Midi Kit (Macherey-Nagel, Düren, Germany) according to the manufacturer's manual.

### Standard cellculture

HEK2₉₃ FT cells were cultivated at 37 °C with 5 % CO₂ at saturated humidity using Dulbecco's Modified Eagle's Medium (DMEM) (25 mM glucose, 1mM Sodium Pyruvate) (Sigma Aldrich, Taufkirchen, Germany), supplemented with 10 % Fetal Bovine Serum (FCS) (heat-deactivated) (VWR, Darmstadt, Germany), 100 U/ml Penicillin, 10 µg/ml Streptomycin (P/S) (Sigma Aldrich) and 2 mM L-Glutamine (Sigma Aldrich) (standard medium).

Passaging was carried out by removing old medium, washing with Phosphate Buffered Saline (PBS) (Sigma Aldrich), incubating 5 min at 37 °C in Trypsin (EDTA-solution) (Sigma Aldrich), pelleting the cells by centrifugation at 300 g for 5 min and dissolving the pellet in new standard medium. The needed amount of cells was approximated or calculated following counting in a Neubauer chamber, suspended in the needed amount of standard medium for cultivation in a Falcon tube and transferred to the cell culture dish.

For freezing 2.5*10⁵ to 2*10⁶ cells were pelleted as in passaging, diluted in 1 ml of Cryomedium (standard-medium supplemented with 10 % dimethyl sulfoxide (DMSO) (Sigma Aldrich)), transferred to a cryo-tube and stored at -86 °C in a styrofoam box. For long-term storage cells were transferred to liquid nitrogen later on.

To thaw cells the cryo tube was placed in a 37 °C water bath right until the medium completely reached the liquid stage. The content of the cryo-tube was mixed with 5 ml medium and centrifuged for 5 min at 300 g. The supernatant was discarded and the pellet was resuspended in fresh standard medium upon transfer to a cell culture dish.

Cells were checked for Mycoplasma contamination DAPI-staining on a regular basis. For this, cells were seeded on coverslips and cultivated until near confluency. They were washed with PBS and fixed 20 min at room temperature with paraformaldehyde (PFA) (4 %) (Roth, Karlsruhe, Germany). After washing three times for 5 min with PBS the cells were incubated 15 min with 4,6-diamidino-2-phenylindole (DAPI) (1 µg/ml in PBS) (Sigma Aldrich) under the exclusion of light. After washing two times with PBS for 5 min, samples were rinsed shortly with ddH₂O before mounting them using Mowiol 4-88 (Roth). Imaging was performed using fluorescence microscopy.

### Creation of knockout-clones

First a homozygous IKK2-knockout-clone was created, the the IKK1-knockout-design was applied to it to create the double.knockout.

The cells were transfected using calcium-phosphate precipitation. Cells were seeded in a 6-well-plate-well and incubated in standard medium overnight. 1.25 µg of each vector were put together and ddH₂O was added until 60 µl of total volume were reached. 60 µl 2x HEPES buffered saline (HBS) (lab-made) were added and the solution was vortexed. 6 µl calciumchloride (CaCl₂) (2.5 M) (Roth) were added, the solution was inverted several times and incubated for 25 min at room temperature. Small drops of the solution were given to the medium, the cells were carefully shaken in an 8-shaped manner and incubated for 6 h at 37 °C before the medium was changed to standard medium. The following day the medium was changed to standard medium with Puromycin (1 µg/ml) (Sigma Aldrich). After 5 days, the medium was changed to standard medium again.

Single cell dilution was performed according to Maldonado and Melendez-Zaigla (Maldonado, Melendez-Zaigla, 2007). The wells were screened and wells with only one cell were incubated until colonies had formed which were passaged to 24-well-plates, by washing them with PBS (Sigma Aldrich), detaching them with Trypsin (Sigma Aldrich), relocating them and adding standard medium. Once the cells in a well reached confluency, they were passaged to two 12-well-wells in the same manner. After reaching confluency again, one well was lysed to generate a DNA template for a genomic PCR whereas the other was passaged to two 6-well-wells, which were frozen when the cell count reached roughly 10⁶ cells.

### Genomic PCR

To create a DNA-template cells were harvested as for passaging, but the pellet was resuspended in roughly 200 µl lysis-buffer (lab-made) per 10⁶ cells, supplemented with protein kinase K (Serva Electrophoresis, Heidelberg, Germany) (200 ng/ml final concentration) and incubated at 55 °C for 1 h under fast shaking, followed up by 5 min at 95 °C. PCR was carried out according to the following scheme and the fragments were analysed per gel electrophoresis using 2 % agarose-gel with TAE-buffer (lab-made) and EtBr (Roth).

**Table 3: Reaction scheme Genomic PCR. Negative-control using H₂O instead of template.**

| Genomic PCR | |
|---|---|
| Component | Quantity [µl] |
| ddH₂O | 35.75 |
| 5x GoTaq Buffer (Promega) | 10 |
| GoTaq polymerase (Promega) | 0.25 |
| Deoxynucleoside triphosphates (dNTPs) (10 mM) (New England Biolabs) | 1 |
| forward primer 10 µM | 1 |
| reverse primer 10 µM | 1 |
| DNA-template | 1 |

**Table 4: Thermocycler-program for Genomic PCR.**

| Thermocycler program Genomic PCR | | |
|---|---|---|
| Repetitions | Time [sec] | Temperature [°C] |
| 1X | 180 | 94 |
| 35^{X} | 30 | 94 |
| | 30 | 59 |
| | 60 | 72 |
| 1X | 300 | 72 |

### RNA isolation and cDNA synthesis

Cell pellets of 10⁶ cells (harvested as in passaging) were lysed in 1 ml Trizol (TRIReagent) (Sigma Aldrich) by pipetting up and down and incubating for 5 min at room temperature. 0.2 ml chloroform (Roth) were added and the samples were shaken thoroughly before incubation for 15 min at room temperature and following centrifugation for 15 min at 12000 g and 2 °C. The watery supernatant was transferred to a new reaction tube, mixed with 0.5 ml isopropanol (chemical storage), incubated 10 min at room temperature, centrifuged 10 min at 12000 g and 2 °C and the new supernatant was removed. The pellet was vortexed with 1 my 75 % Ethanol (chemical storage) before the samples were centrifuged for 5 min at 12000 g and 2 °C. The supernatant was removed again and the pellet was dried at room temperature before it was resuspended in 20 µl RNAse-free-water (lab-made) by pipetting up and down and shaking for 15 min at 60 °C. The RNA-concentration was measured using a NanoDrop 1000 Spectrophotometer (Thermo Fisher Scientific, Waltham, USA). To purify the RNA an ammonium acetate precipitation was performed by adding 20 µl ammonium-acetate (NH₄-acetate) (4M) (Roth) and 25 µl Isopropanol (chemical storage) and vortexing the sample before incubation for 5 min at room temperature and following centrifugation for 15 min at 12000 g and room temperature. The supernatant was removed and 200 µl 70 % Ethanol (chemical storage) were added to the samples right before centrifugation for 5 min at 12000 g and room temperature. The supernatant was removed again and the pellets were dried at room temperature and suspended in 20 µl RNAse free water (lab-made) in the same manner as before. The concentration was quantified using a NanoDrop 1000 Spectrophotometer (Thermo Fisher Scientific) again.

DNAse digestion was performed by using 1 µg of the RNA per sample and raising the volume to 8 µl by using RNAse free water (lab-made), when 1 µl RQ1 RNase-free DNase 10x Reaction buffer (Promega) and 1 µl RQ1 RNase free DNase (Promega) were added. The samples were incubated for 30 min at 37 °C upon 1 µl RQ1 DNase stop-solution (Promega, Madison, USA) was added with subsequent incubation at 65 °C for 10 min.

1 µl random primer (Promega) and 3 µl RNAse-free water (chemical storage) were added, the samples were incubated for 5 min at 70 °C with consecutive cooling on ice, before cDNA first strand synthesis was carried out using the following scheme (Tabi) with eventual incubation for 60 min at 42 °C.

**Table 5: Reaction scheme cDNA first strand synthesis.**

| cDNA first strand synthesis | |
|---|---|
| Component | Quantity [µl] |
| RNA-template | 15 |
| Deoxynucleoside triphosphates (dNTPs) (10 mM) (New England Biolabs) | 1.25 |
| RNasin Ribonuclease Inhibitor (Promega) | 0.6 |
| Moloney murine leukemia virus 5x Reaction Buffer (M-MLV-buffer) (Promega) | 5 |
| Moloney murine leukemia virus reverse transcriptase (M-MLV RT) (Promega) | 1 |
| RNase free water (lab-made) | 2.15 |

Following a PCR was performed according to the genomic PCR scheme using cDNA as template, with GAPDH-Primers as control.

### Western blot

Cells were either washed with PBS (Sigma Aldrich), detached with Trypsin (Sigma Aldrich) and pelleted, or washed with PBS (Sigma Aldrich) and then lysed in the plate. The lysis buffer (RIPA-buffer) (lab-made) supplemented with 10 % proteinase-inhibitor (1.54 µM Aprotinin) (Roth) and 1 % phosphatase-inhibitor (Halt™ Phosphatase Inhibitor Cocktail) (Thermo Fisher Scientific) was added and in the case of in-plate-lysis the cells were detached with a cell-scraper. The cells were lysed with a pipette until a homogenous suspension was reached, which was centrifuged at 13000 rpm for 1 min. The protein-concentration of the supernatant was determiQed with a Nanodrop 1000 Spectrophotometer (Thermo Fisher Scientific) or BCA-Assay (BCA™ Protein Assay Kit, Prod# 23225, Pierce, Thermo Fisher Scientific; Photometer: GloMax®-Multi Detection System, Promega). The samples were diluted to contain 40 µg protein per 30 µl and 10 µl 4x SDS-PAGE loading buffer (lab-made) were added before 3 min incubation at 95 °C. Two gels were manufactured according to Tab 7 and 8. The gels were run using SDS-PAGE-buffer (lab-made) and 80 V were applied during the stacking gel-phase whereas 30 mA were applied during the running gel-phase.

**Table 6:10 % polyacrylamide gel composition.**

| 10 % polyacrylamide running gel | |
|---|---|
| Component | Quantity |
| Acrylamide-Bisacrylamide (40% w/v), (Serva-Electrophoresis) | 4 ml |
| Tris(hydroxymethyl)-aminomethan (TRIS) (Roth) 3 M, pH 8.8 | 2 ml |
| Sodium dodecyl sulfate (SDS) (chemical storage) 10 % | 160 µl |
| ddH₂O | 9.73 ml |
| Tetramethylethylenediamine (TEMED) (Roth) | 13.3 µl |
| Ammonium persulfate (APS) (Thermo Fisher Scientific) 10 % | 133 µl |

**Table 7: 3.75 % polyacrylamide stacking gel composition.**

| SDS-PAGE stacking gel | |
|---|---|
| Component | Quantity |
| Acrylamide-Bisacrylamide (40% w/v), (Serva-Electrophoresis) | 375 µl |
| Tris(hydroxymethyl)-aminomethan (TRIS) (Roth) 1 M pH 8.8 | 500 µl |
| Sodium dodecyl sulfate (SDS) (chemical storage) 10 % | 40 µl |
| ddH₂O | 2.06 ml |
| Tetramethylethylenediamine (TEMED) (Roth) | 3.5 µl |
| Ammonium persulfate (APS) (Thermo Fisher Scientific) 10 % | 35 µl |
| Saccharose (Roth) 60 % | 980 µl |

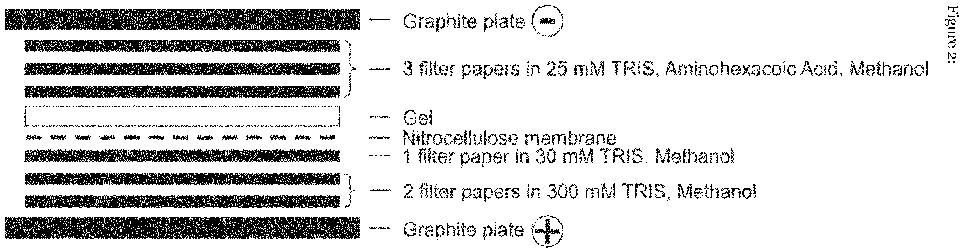

After running the SDS-PAGE the gels were placed in a blotting-apparatus and an Immunoblot according to Fig. 2 was performed to transfer the protein to a nitrocellulose membrane. A current intensity of 260 mA was applied for 1.5 h.

Following electrotransfer, the membrane was washed in H₂O, stained (5 % Ponceau S (Roth) in H₂O), again washed in H₂O and pictured. It was decolorized by washing in H₂O with 3-5 drops 2 M Tris(hydroxymethyl)-aminomethan (TRIS) (Roth) in H₂O. The Membrane was blocked overnight at 4 °C in washing solution (lab-made) supplemented with 5 % milk powder (Roth) and subsequently washed (3x 10 min) in washing solution (lab-made). It was incubated with the primary antibody solution (either anti IKKα (M-280): sc-7182 rabbit (Santa Cruz Biotechnology, Dallas, USA) 1:500 or anti IKKβ rabbit (#2370) (Cell Signaling, Danvas, Texas, USA) 1:1000 in washing solution (lab-made)) for 2.5 h at room temperature, washed (3x 10min) in washing solution (lab-made) and incubated with the secondary antibody (horseradish peroxidase-conjugated sheep anti-rabbit, (Dianova, Hamburg, Germany), 1:4000 in washing solution (lab-made)) for 1 h at room temperature. It was washed (3x 10 min) in washing solution (lab-made) and (1x 10 min) in 50 mM Tris(hydroxymethyl)-aminomethan-hydrochloride (TRIS-HCl) (TRIZMA®hydrochloride) (Sigma Aldrich) pH 7.5, before the enhanced chemiluminescence (ECL) reaction was used for protein detection. A mix of 4 ml solution A (lab-made) and 400 µl solution B (lab-made) was poured onto the membrane and a Super RX-N Fuji Medical X-Ray Film (Fujifilm, Tokio, Japan) was exposed (different time frames used to optimize), developed in GBX-Developer (Carestream, New York, USA), washed with H₂O, fixed with 'Fixier Konzentrat' (Adefo-Chemie, Neu-Isenburg, Germany) and washed in H₂O again. A loading control was performed using GAPDH in the same manner with mouse anti GAPDH (Santa Cruz Biotechnology) (1:4000) as primary antibody and horseradish peroxidase-conjugated sheep anti-mouse (Dianova) (1:4000) as secondary antibody. X-Ray films were scanned (Epson perfection 4990 PHOTO, Epson, Suwa, Japan) and band intensity was measured using Fiji, open source {Schindelin, 2012 #4}.

### Flow-cytometry

Cells were seeded in 6-well-plate wells overnight and medium was changed to standard medium supplemented with Tumor Necrosis Factor α (TNFα) (Calbiochem®, Human, Recombinant, *E. coli*) (Merck, Darmstadt, Germany) at 20 ng/ml. The cells were incubated overnight at 37 °C and 5 % CO₂, pictures were taken, the medium was removed and transferred to a falcon tube, the cells were washed with PBS (Sigma Aldrich), also taken to the falcon tube and incubated for 5 min in Trypsin (Sigma Aldrich) in which the cells were detached and which was taken to the falcon tube as well. After centrifugation at 300 g for 5 min the supernatant was removed and the pellet was washed with 1 ml 70 % Ethanol (chemical storage) for 5 min. 2 ml Staining Solution (lab-made) were added and the samples were incubated for 60 min at 37 °C. The samples were filtered using 22 µm filters (Sar-stedt, Numbrecht, Germany) and the staining was analysed using flow cytometry (Gallios™ Flow Cytometer, Beckman Coulter, Indianapolis, USA) and the Software Kaluza 1.2 (http://www.beckmancoulter.de/Kaluza.html).

Sequences of sgRNA and primer are shown in table 8:

**Table 8: sgRNA and Primer Sequences**

| **Name** | **sgRNA Sequences shown as DNA*** | **SEQ ID NO:** |
|---|---|---|
| IKK1_sgRNA1 | 5' GTGTGTGTCTCTGTGAATG 3' | 1 |
| IKK1_sgRNA2 | 5' GTCACACTCACTAAACACC 3' | 2 |
| IKK1_sgRNA3 | 5'GCCGGGCGCGGGCGGGCCCT3' | 3 |
| IKK1_sgRNA4 | 5' AGCCCCGGGGGCCGCTCCA 3' | 4 |
| IKK2_sgRNA1 | 5' TGGTGCTGTGAGTGGGGCT 3' | 5 |
| IKK2_sgRNA2 | 5' ACCGCAATGCCTGGGTCAG 3' | 6 |
| IKK2_sgRNA3 | 5' GCTGACCCACCCCAATGTGG 3' | 7 |
| IKK2-sgRNA4 | 5' ATCCCCTCAGGGACATCTC 3' | 8 |

| | **DNA primer for diagnostic** | |
|---|---|---|
| IKK1_KO_fwd | 5' G CCTTGG AACAACTGTGGAAC 3' | 9 |
| IKK1_KO_rev | 5' GG ACGCACACATTCCCAAAG 3' | 10 |
| IKK2_KO_fwd | 5' CCACCTTGTTTGCCTGCTTG 3' | 11 |
| IKK2_KO_rev | 5' GTCTCCCCATCCCTCTTCCT 3' | 12 |

| | | |
|---|---|---|
| *The sequences of RNA molecules are provided as DNA for convenience. RNA molecules contain a uracil base instead of thymidine. | | |

### Example 1: Design and Creation of IKK1 and IKK2 Knock-Outs Using CRISPR

To create IKK1-knockout-cell-lines the designed Oligonucleotides each were cloned into pX462 vectors. Wildtype (WT) and IKK2^{-/-}-cells were transfected with all four corresponding vectors. To optimize efficiency two different designs were created and compared. With the mixed culture of the more efficient design a single cell cloning assay was carried out to obtain homozygous knockout-clones. Knockouts were characterised on DNA-level by genomic PCR, on mRNA-level by RT-PCR and following PCR with one primer annealing in the desired deletion and on protein-level by Western blotting. Verified knockout clones (IKK.2^{-/-}, IKK1^{-/-} and IKK2^{-/-}/IKK1^{-/-}) were tested for their reaction to TNFα-stimulation via propidium iodide (PI) flow cytometric assay.

Wildtype (WT)- and IKK2^{-/-}-cells were transfected with all four selected vectors of one design simultaneously using calcium phosphate precipitation {Chen, 1987 #5}, selected with puromycin for 5 days. A single cell cloning assay was performed to obtain homozygous knockout-clones of the more efficient second design.

Based on the Genomic PCR, homozygous clones were chosen (Fig. 3A). Successful knockouts were validated on mRNA level using primer pairs, in which one primer was designed within the desired deletion (Fig. 3B). Finally, absence of IKK1 and IKK2 protein was confirmed with Western blotting (Fig. 3C).

The genomic PCR shows the expected pattern with all IKK2^{-/-}-cells showing knockout-bands at use of IKK₂-diagnostic Primers as all IKKn1^{-/-}cells do at use of IKK1-diagnostic primers (Fig. 1A). The RT-PCR only shows bands in the cells without corresponding knockout, confirming the absence of intact mRNA in those with (Fig. 1B). The western blot clearly shows absence of IKK2 protein in cells without IKK2-knockout and presence of IKK1 protein only in wildtype and IKK2-single-knockout-cells (Fig. 1C).

### Example 2: characterization of IKK1 and IKK2 Double Mutants

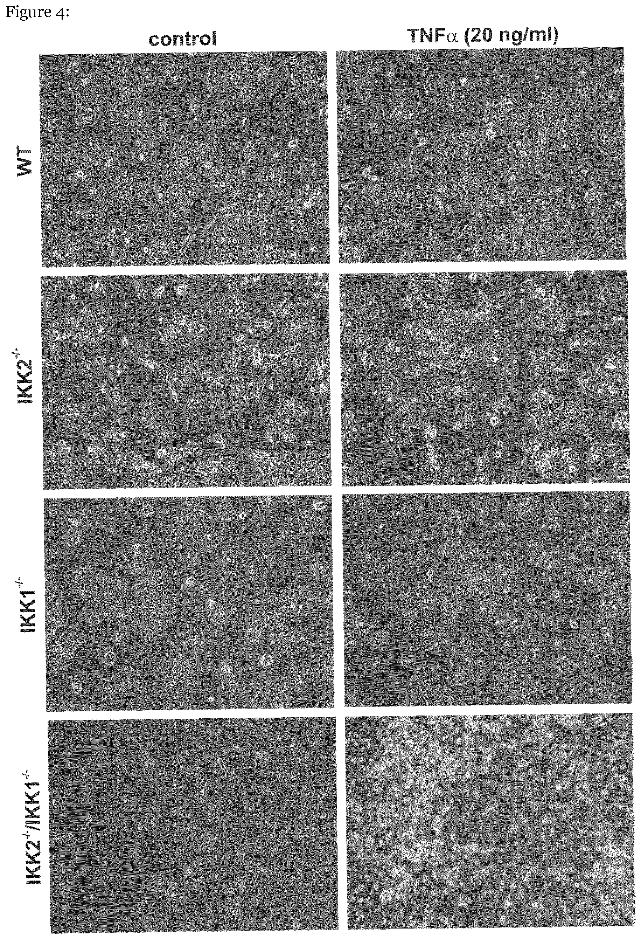

Clones were -stimulated with TNFα to observe the cells reaction. About 20 h after stimulation pictures were taken (Fig. 4). No apparent effect of TNFα stimulation for single-knockouts and wildtype was observable but a severely increased number of dead cells could be noted for the stimulated double-knockout.

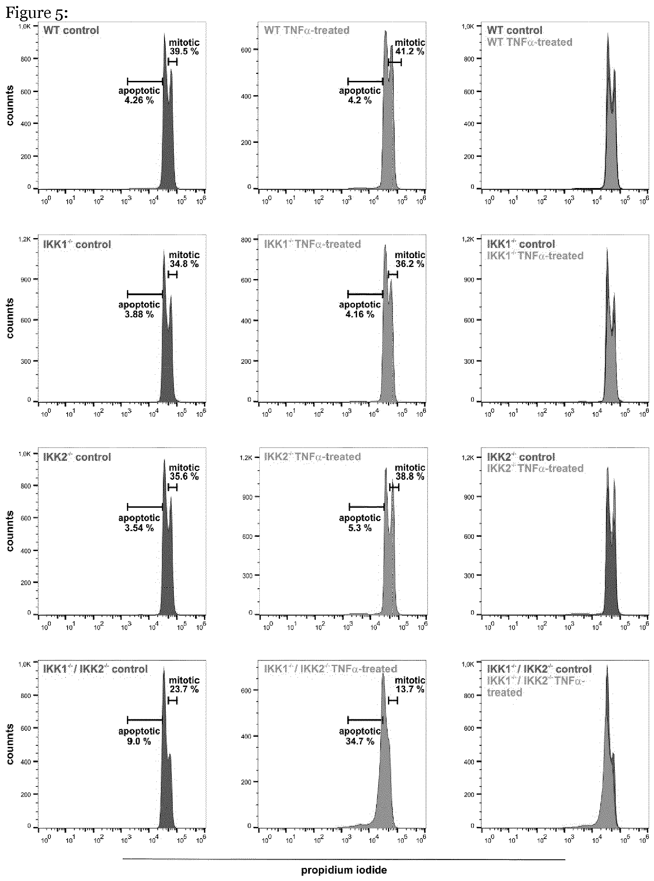

For more precise evaluation of apoptosis a propidium iodide (PI) flow cytometric assay was performed. The obtained results are shown in Fig. 5.

About the same count of apoptotic and mitotic cells was measured in unstimulated wildtype and single-knockouts. The apoptotic count ranged from 3,54 % to 4,26 % and the mitotic count from 34.8 % to 39.5 %, with both the apoptotic and the mitotic count being the highest in wildtype. The unstimulated double-knockout clearly differed with 9 % apoptotic and 23.7 % mitotic cells.

Upon stimulation the apoptotic, as well as the mitotic count in the single-knockouts raised. The apoptotic count ranged from 4.16 % to 5.3 % and the mitotic count from 36.2 % to 38.8 %. In wildtype-cells the apoptotic count seemed to drop minimally, while the mitotic count raised a little.

The double-knockout cells showed drastic changes upon stimulation. The apoptotic count raised to 34.7 %, thus is almost four times higher than for unstimulated cells and the mitotic count diminished to 13.7 %. The overlay clearly shows the absence of a mitotic peak.

## Claims

1. An antagonist of IKK1 and/or an antagonist of IKK2 for use in a method of treating a proliferative disorder in a subject, the method comprising the step of reducing or impairing the function or expression of both IKK1 and IKK2 simultaneously in a cell which is associated with the proliferative disorder in the subject.

2. The antagonist for use according to claim 1, wherein the proliferative disorder is a tumor disease, such as ovarian cancer, and said cell associated with the proliferative disorder in the subject is a tumor cell.

3. The antagonist for use according to claim 1 or 2, wherein reducing or impairing the function or expression of both IKK1 and IKK2 simultaneously in a cell involves gene editing, gene silencing (RNA interference) or small molecule inhibitors of IKK1 and IKK2.

4. The antagonist for use according to any one of claims 1 to 3, wherein reducing or impairing the function or expression of both IKK1 and IKK2 simultaneously in a cell involves gene editing comprising the introduction of single guide RNA(s) into the cell for targeting the genes of IKK1 and IKK2, and wherein said antagonist of IKK1 and/or the antagonist of IKK2 is an sgRNA specific for IKK1/IKK2.

5. The antagonist for use according to claim 4, further comprising the introduction of a gene editing nuclease into the cell, for example by introducing into the cell an expressible DNA or RNA construct encoding for the gene editing nuclease, or introducing into the cell a gene editing nuclease protein.

6. The antagonist for use according to claim 5, wherein the gene editing nuclease is selected from any gene editing compatible DNA nuclease such as Cpfl or Cas9, or an optimized nuclease such as Cas9-HF1 or eSpCas9, but preferably is a nickase, such as Cas9 D10A and Cas9 H840A.

7. The antagonist for use according to any one of claims 1 to 6, wherein the antagonist of IKK1 is a sgRNA selected from SEQ ID NO: 1 to 4 and/or wherein the antagonist of IKK2 is a sgRNA selected from any one of SEQ ID NO: 5 to 8.

8. The antagonist for use according to any one of claims 4 to 7, wherein said gRNA, or gRNA expression construct, or said gene editing nuclease, or gene editing nuclease expressing construct, is encapsulated in a viral particle or a nanoparticle for cellular delivery.

9. The antagonist for use according to any one of claims 1 to 8, wherein the method comprises a step of activating Tumor Necrosis Factor (TNF) mediated signaling in the cell associated with the disease.

10. The antagonist for use according to claim 9, wherein activating Tumor Necrosis Factor (TNF) mediated signaling in the cell associated with the disease comprises TNF receptor (TNFR)-activation, such as TNFR1 and/or TNFR2, for example by using TNFα, a TNFα analog, or a TNFR agonist.

11. A composition for use in a method for treating a proliferative disorder in a subject, the composition comprising an inhibitor/antagonist of IKK1 and an inhibitor/antagonist of IKK2.

12. The composition for use according to claim 11, for use in a method according to any one of claims 1 to 10.

13. The composition for use according to claim 11 or 12, wherein the inhibitor of IKK1 and/or the inhibitor of IKK2, is a nucleic acid compound, preferably an RNA-compound, such as a therapeutic gRNA.

14. The composition for use according to claim 13, comprising at least two pairs of gRNA, wherein the first pair targets IKK1 and the second pair targets IKK2 to introduce loss-of-function mutations into the genes of IKK1 and IKK2 by gene editing.

15. The composition for use according to any one of claims 11 to 14, further comprising administering to the subject a gene editing nuclease, preferably in an amount sufficient to effectively induce gene editing in a target cell in the presence of sgRNA.


SEQUENCE LISTING

<110>  Universitaet Bielefeld

<120>  DUAL INHIBITION OF IKK1 AND IKK2 FOR THE TREATMENT OF
       PROLIFERATIVE DISEASES

<130>  U30791EP

<160>  12

<170>  PatentIn version 3.5

<210>  1
<211>  19
<212>  DNA
<213>  Artificial

<220>
<223>  guide RNA

<400>  1
gtgtgtgtct ctgtgaatg                                                    19


<210>  2
<211>  19
<212>  DNA
<213>  artificial

<220>
<223>  guide RNA

<400>  2
gtcacactca ctaaacacc                                                    19


<210>  3
<211>  20
<212>  DNA
<213>  artificial

<220>
<223>  guide RNA

<400>  3
gccgggcgcg ggcgggccct                                                   20


<210>  4
<211>  19
<212>  DNA
<213>  artificial

<220>
<223>  guide RNA

<400>  4
agccccgggg gccgctcca                                                    19


<210>  5
<211>  19

<212>  DNA
<213>  artificial

<220>
<223>  guide RNA

<400>  5
tggtgctgtg agtggggct                                                    19


<210>  6
<211>  19
<212>  DNA
<213>  artificial

<220>
<223>  guide RNA

<400>  6
accgcaatgc ctgggtcag                                                    19


<210>  7
<211>  20
<212>  DNA
<213>  artificial

<220>
<223>  guide RNA

<400>  7
gctgacccac cccaatgtgg                                                   20


<210>  8
<211>  19
<212>  DNA
<213>  artificial

<220>
<223>  guide RNA

<400>  8
atcccctcag ggacatctc                                                    19


<210>  9
<211>  21
<212>  DNA
<213>  artificial

<220>
<223>  primer

<400>  9
gccttggaac aactgtggaa c                                                 21


<210>  10
<211>  20
<212>  DNA
<213>  artificial

<220>
<223>  primer

<400>  10
ggacgcacac attcccaaag                                                    20


<210>  11
<211>  20
<212>  DNA
<213>  artificial

<220>
<223>  primer

<400>  11
ccaccttgtt tgcctgcttg                                                    20


<210>  12
<211>  20
<212>  DNA
<213>  artificial

<220>
<223>  primer

<400>  12
gtctccccat ccctcttcct                                                    20